(19) **Europäisches Patentamt**
**European Patent Office**
**Office européen des brevets**

(11) **EP 0 948 572 B2**

(12) **NEUE EUROPÄISCHE PATENTSCHRIFT**
Nach dem Einspruchsverfahren

(45) Veröffentlichungstag und Bekanntmachung des Hinweises auf die Entscheidung über den Einspruch:
**20.01.2010 Patentblatt 2010/03**

(45) Hinweis auf die Patenterteilung:
**16.07.2003 Patentblatt 2003/29**

(21) Anmeldenummer: **97923993.6**

(22) Anmeldetag: **23.05.1997**

(51) Int Cl.:
**C09C 1/00** (2006.01)   **C09D 7/12** (2006.01)
**C09D 11/00** (2006.01)   **C08K 3/00** (2006.01)
**C04B 33/14** (2006.01)   **C03C 4/02** (2006.01)

(86) Internationale Anmeldenummer:
**PCT/EP1997/002651**

(87) Internationale Veröffentlichungsnummer:
**WO 1998/053011 (26.11.1998 Gazette 1998/47)**

(54) **MEHRSCHICHTIGE INTERFERENZPIGMENTE**

MULTI-COATED INTERFERENCE PIGMENTS

PIGMENTS DE POLARISATION MULTICOUCHES

(84) Benannte Vertragsstaaten:
**DE ES FI FR GB IT**

(43) Veröffentlichungstag der Anmeldung:
**13.10.1999 Patentblatt 1999/41**

(73) Patentinhaber: **Merck Patent GmbH**
**64271 Darmstadt (DE)**

(72) Erfinder:
• **BRÜCKNER, Hans-Dieter**
**D-64289 Darmstadt (DE)**
• **HEYLAND, Andrea**
**D-64385 Reichelsheim (DE)**
• **SCHMIDT, Christoph**
**D-65830 Kriftel (DE)**
• **SCHANK, Christina**
**D-64372 Ober-Ramstadt (DE)**
• **SEIBEL, Claudia**
**D-64853 Otzberg (DE)**

(56) Entgegenhaltungen:
**EP-A1- 0 370 701    EP-A1- 0 632 109**
**EP-A1- 0 803 552    EP-A2- 0 708 154**
**EP-A2- 0 718 378    EP-A2- 0 753 545**
**WO-A1-93//08237    WO-A1-94//01498**
**WO-A1-98//12266    DE-A1- 19 618 569**
**JP-A- 06 093 206    JP-A- 07 246 366**
**US-A- 3 553 001**

• **DATABASE WPI Week 9547 Derwent Publications Ltd., London, GB; AN 95-363063 XP002051685 & JP 07 246 366 A (MATSUDA) , 26.September 1995**
• **DATABASE WPI Week 9009 Derwent Publications Ltd., London, GB; AN 90-063308 XP002051513 & JP 02 016 168 A (KANEBO LTD) , 19.Januar 1990**
• **DATABASE WPI Week 9413 Derwent Publications Ltd., London, GB; AN 94-106716 XP002051514 & JP 06 056 628 A (POLA CHEM. IND. INC.) , 1.März 1994**
• **CHEMICAL ABSTRACTS, vol. 110, no. 20, 15.Mai 1989 Columbus, Ohio, US; abstract no. 176060y, TSUGITA, AKIRA ET AL.: "Preparation of titania-alumina doubly coated mica powders and their optical properties" Seite 165; XP000056533 & SHIKIZAI KYOKAISHI, Bd. 61, Nr. 12, 1988, JAPAN, Seite 685-691**
• **DR. RALF GLAUSCH ET AL: 'Perlglanzpigmente', 1996, DR. ULRICH ZORLL, HANNOVER Artikel 'Herstellung und Eigenschaften von Perglanzpigmenten', Seiten 11 - 43**
• **C.SCHMIDT ET AL: 'Optical Physics of Synthetic Interference Pigments' KONTAKTE 1992, DARMSTADT, Seiten 15 - 24**
• **Artikel (FIG 13): 'The causes and prevention of titanium dioxide phototdegradation of paints', Seite 112**

**EP 0 948 572 B2**

**Beschreibung**

Die Erfindung betrifft mehrschichtige Interferenzpigmente, bestehend aus einem transparentem Trägermaterial, das mit alternierenden Schichten eines Metalloxides mit niedriger Brechzahl und eines Metalloxides mit hoher Brechzahl beschichtet ist.

Mehrschichtige Pigmente mit niedriger Transparenz und ähnlichem Schichtaufbau sind bekannt. Die Metalloxidschichten werden entweder im Naßverfahren durch Auffällen der Metalloxidhydrate aus einer Metallsalzlösung auf ein Trägermaterial oder durch Aufdampfen oder Sputtern im Vakuum hergestellt. Generell sind die Aufdampfverfahren für eine Massenproduktion von Pigmenten zu aufwendig und kostspielig. So beschreibt US 4 434 010 ein mehrschichtiges Interferenzpigment, bestehend aus einer zentralen Schicht eines reflektierenden Materials (Aluminium) und alternierenden Schichten zweier transparenter, dielektrischer Materialien mit hoher und niedriger Brechzahl, beispielsweise Titandioxid und Siliziumdioxid beiderseits der zentralen Aluminiumschicht. Dieses Pigment wird für den Druck von Wertpapieren eingesetzt.

JP H7-759 (Kokoku) beschreibt ein mehrschichtiges Interferenzpigment mit metallischem Glanz. Es besteht aus einem Substrat, das mit alternierenden Schichten von Titandioxid und Siliziumdioxid beschichtet ist. Das Substrat wird aus Aluminium-, Gold- oder Silberplättchen oder Plättchen aus Glimmer und Glas, die mit Metallen beschichtet sind, gebildet. Es handelt sich demnach um ein typisches Metalleffektpigment. Dieses Pigment besitzt hohes Deckvermögen. Für Anwendungen, bei denen eine hohe Transparenz des pigmentierten Materials gefordert wird, beispielsweise für Agrarfolien, ist das Pigment ungeeignet. Weiterhin hat es den Nachteil, daß der typische Tiefeneffekt von Interferenzpigmenten nicht erzeugt wird, da durch die hohe Reflektion des Lichtes an der den Kern bildenden Metallschicht, tiefer im Anwendungsmedium liegende Pigmentteilchen nicht zum optischen Erscheinungsbild beitragen können. Der Interferenzeffekt bleibt deshalb auf die sich auf der Metallschicht befindenden Schichten begrenzt.

EP 0 708 154 beschreibt Glanzpigmente aus beschichteten, plättchenförmigen, metallischen Substraten. Die Beschichtung besteht aus farblosen Schichten mit einem Brechungsindex $n \leq 1,8$ und selektiv absorbierenden Schichten mit einem Brechungsindex $n \geq 2,0$.

In der JP-A-07-246 366 wird ein optisches Interferenzmaterial beschrieben, das aus alternierenden hochbrechenden und niedrigbrechenden Schichten aufgebaut ist, wobei die optische Dicke jeder Schicht ein ungeradzahliges Vielfaches eines Viertels der zu interferierenden Lichtwellenlänge beträgt.

Aus der JP-A-02-016 168 ist ein pulverförmiges Farbpigment mit einer Titanoxidschicht, einer Aluminiumoxidschicht sowie einer Schicht aus einem gefärbten anorganischen Material bekannt.

Aufgabe der Erfindung ist es, ein im wesentlichen transparentes Interferenzpigment mit kräftigen Interferenzfarben und/oder einer starken Winkelabhängigkeit der Interferenzfarben zur Verfügung zu stellen. Darüber hinaus besteht die Aufgabe der Erfindung darin, Pigmente mit speziellen, spektralen Charakteristiken im sichtbaren Bereich und im Infrarot-Bereich zur Verfügung zu stellen.

Diese Aufgabe wird gemäß Anspruch 1 gelöst.

Das transparente Trägermaterial ist Glimmer, ein anderes Schichtsilikat, Glasplättchen, $PbCO_3$ x Pb $(OH)_2$ sowie BiOCl in Plättchenform oder plättchenförmiges Siliziumdioxid, hergestellt nach dem in WO 93/08237 beschriebenen Verfahren.

Bei dem Metalloxid mit hoher Brechzahl handelt es sich um ein Oxid oder Mischungen von Oxiden ohne absorbierende Eigenschaften, wie z.B. $TiO_2$, $ZrO_2$ oder ZnO.

Das Metalloxid mit niedriger Brechzahl ist $SiO_2$, $Al_2O_3$, AlOOH, $B_2O_3$ oder eine Mischung daraus und kann ebenfalls absorbierende oder nicht absorbierende Eigenschaften haben. Gegebenenfalls kann die Oxidschicht mit niedriger Brechzahl Alkali- und Erdalkalioxide als Bestandteile enthalten.

Weiterhin wird diese Aufgabe gemäß der Erfindung durch ein Verfahren zur Herstellung der erfindungsgemäßen Pigmente gelöst, indem das transparente Trägermaterial in Wasser suspendiert und abwechselnd mit einem Metalloxidhydrat mit hoher Brechzahl und einem Metalloxidhydrat mit niedriger Brechzahl durch langsame Zugabe und Hydrolyse der entsprechenden, anorganischen, wasserlöslichen Metallverbindungen beschichtet wird, wobei durch gleichzeitige Zugabe von Säure oder Base der für die Fällung des jeweiligen Metalloxidhydrates notwendige pH-Wert eingestellt und konstant gehalten wird und wobei zumindest die Schichtfolge hochbrechendes Metalloxid / niedrigbrechendes Metalloxid /hochbrechendes Metalloxid aufgefällt wird und anschließend das beschichtete Trägermaterial aus der wäßrigen Suspension abgetrennt, getrocknet und gegebenenfalls kalziniert wird.

Gegenstand der Erfindung ist außerdem die Verwendung der erfindungsgemäßen Pigmente zur Pigmentierung von Lacken, Druckfarben, Kunststoffen, Glasuren für Keramiken und Gläser, Kosmetika und insbesondere zur Herstellung von Agrarfolien.

Hierfür können sie als Mischungen mit handelsüblichen Pigmenten, beispielsweise anorganischen und organischen Absorptionspigmenten, Metalleffektpigmenten und LCP-Pigmenten, eingesetzt werden.

Die Dicke der Schichten der Metalloxide mit hoher und niedriger Brechzahl ist wesentlich für die optischen Eigenschaften des Pigmentes. Da ein Produkt mit kräftigen Interferenzfarben erwünscht ist, muß die Dicke der Schichten aufeinander eingestellt werden. Wenn n die Brechzahl einer Schicht und d ihre Dicke ist, ergibt sich die Interferenzfarbe, in der eine dünne Schicht erscheint, aus dem Produkt von n und d, d.h. der optischen Dicke. Die bei Normallichteinfall im reflektierenden Licht entstehenden Farben eines solchen Filmes ergeben sich aus einer Verstärkung des Lichtes der Wellenlänge

$$\lambda = \frac{4}{2N-1} \cdot nd$$

und durch Schwächung von Licht der Wellenlänge

$$\lambda = \frac{2}{N} \cdot nd,$$

wobei N eine positive ganze Zahl ist. Die bei zunehmender Filmdicke erfolgende Variation der Farbe ergibt sich aus der Verstärkung bzw. Schwächung bestimmter Wellenlängen des Lichtes durch Interferenz. Beispielsweise hat ein 115 nm dicker Film aus Titandioxid mit der Brechzahl 1,94 eine optische Dicke von 115 x 1,94 = 223 nm, und Licht der Wellenlänge 2 x 223 nm = 446 nm (blau) wird bei der Reflexion geschwächt, so daß das reflektierte Licht gelb ist. Bei mehrschichtigen Pigmenten wird die Interferenzfarbe durch die Verstärkung bestimmter Wellenlängen bestimmt und wenn mehrere Schichten in einem vielschichtigen Pigment gleiche optische Dicke besitzen, wird die Farbe des reflektierten Lichtes mit zunehmender Zahl der Schichten intensiver und satter. Darüber hinaus kann durch geeignete Wahl der Schichtdicken eine besondere starke Variation der Farbe in Abhängigkeit vom Betrachtungswinkel erreicht werden. Es bildet sich ein ausgeprägter Farbflop aus, der für die Pigmente gemäß der Erfindung erwünscht sein kann. Die Dicke der einzelnen Metalloxidschichten, unabhängig von ihrer Brechzahl beträgt deshalb 50 bis 300 nm.

Die Anzahl und Dicke der Schichten ist abhängig vom gewünschten Effekt und dem verwendeten Substrat. Auf Glimmer erreicht man die gewünschten Effekte, wenn man das 3-Schichtsystem $TiO_2$ / $SiO_2$ / $TiO_2$ aufbaut und die Dicke der einzelnen Schichten optisch aufeinander abstimmt. Bei Verwendung optisch relativ dünner $TiO_2$- und $SiO_2$-Schichten (Schichtdicke < 100 nm) lassen sich z.B. Pigmente mit blauer Interferenzfarbe herstellen, die bei einem wesentlich geringeren $TiO_2$-Gehalt farbkräftiger und transparenter sind als reine $TiO_2$-Glimmerpigmente. Die Einsparung an $TiO_2$ beträgt bis zu 50 Gew.-%.

Durch die Auffällung dicker $SiO_2$-Schichten (Schichtdicke > 100 nm) werden Pigmente mit einer stark ausgeprägten Winkelabhängigkeit der Interferenzfarbe erhalten.

Durch Auffällen weiterer $TiO_2$- und $SiO_2$-Schichten lassen sich auch 5-Schichtsysteme und höhere Systeme erhalten, die Anzahl der Schichten wird aber dann durch die Wirtschaftlichkeit des Pigmentes begrenzt.

Verwendet man jedoch anstelle des Glimmers $SiO_2$-Plättchen einheitlicher Schichtdicke als Substrat, so lassen sich weitere, besonders gut definierte Interferenzeffekte erzielen.

In diesem Fall erhält man durch die Belegung des Substrates mit z.B. 3 Schichten des oben erwähnten Aufbaus ein Interferenzsystem aus 7 dünnen Schichten scharf definierter Dicken. Das Reflexions- bzw. Transmissionsspektrum eines solchen Pigmentes weist feinere und genauer abstimmbare Strukturen auf als das Spektrum eines entsprechenden Pigments, das auf einem Substrat mit breiter Dickenverteilung, z.B. Glimmer, beruht.

Besonders ausgeprägt ist auch die Winkelabhängigkeit der Interferenzfarbe. Dieser extreme Farbflop wird bei konventionellen Metalloxid-Glimmerpigmenten nicht beobachtet.

Die $SiO_2$-Plättchen werden z.B. gemäß der internationalen Anmeldung WO 93/08237 auf einem endlosen Band durch Verfestigung und Hydrolyse einer Wasserglaslösung hergestellt.

Die Metalloxidschichten werden vorzugsweise naßchemisch aufgebracht, wobei die zur Herstellung von Perlglanzpigmenten entwickelten naßchemischen Beschichtungsverfahren angewendet werden können; derartige Verfahren sind z.B. beschrieben in DE 14 67 468, DE 19 59 988, DE 20 09 566, DE 22 14 545, DE 22 15 191, DE 22 44 298, DE 23 13 331, DE 25 22 572, DE 31 37 808, DE 31 37 809, DE 31 51 343, DE 31 51 354, DE 31 51 355, DE 32 11 602, DE 32 35 017 oder auch in weiteren Patentdokumenten und sonstigen Publikationen.

Zur Beschichtung werden die Substratpartikel in Wasser suspendiert und mit einem oder mehreren hydrolysierbaren Metallsalzen bei einem für die Hydrolyse geeigneten pH-Wert versetzt, der so gewählt wird, daß die Metalloxide bzw. Metalloxidhydrate direkt auf den Partikeln niedergeschlagen werden, ohne daß es zu Nebenfällungen kommt. Der pH-Wert wird üblicherweise durch gleichzeitiges Zudosieren einer Base konstant gehalten. Anschließend werden die Pigmente abgetrennt, gewaschen und getrocknet und gegebenenfalls geglüht, wobei die Glühtemperatur im Hinblick auf die jeweils vorliegende Beschichtung optimiert werden kann. Falls gewünscht können die Pigmente nach Aufbringen einzelner Beschichtungen abgetrennt, getrocknet und ggf. geglüht werden, um dann zur Auffällung der weiteren Schichten wieder resuspendiert zu werden.

Weiterhin kann die Beschichtung auch in einem Wirbelbettreaktor durch Gasphasenbeschichtung erfolgen, wobei z.B. die in EP 0,045,851 und EP 0,106,235 zur Herstellung von Perlglanzpigmenten vorgeschlagenen Verfahren entsprechend angewendet werden können.

Als Metalloxid mit hoher Brechzahl wird bevorzugt Titandioxid und als Metalloxid mit niedriger Brechzahl Siliziumdioxid verwendet.

Für das Aufbringen der Titandioxidschichten wird das im US 3 553 001 beschriebene Verfahren bevorzugt.

Zu einer auf etwa 50-100 °C, insbesondere 70-80 °C erhitzten Suspension des zu beschichtenden Materials wird langsam eine wäßrige Titansalzlösung zugegeben, und es wird durch gleichzeitiges Zudosieren einer Base, wie z.B. wäßrige Ammoniaklösung oder wäßrige Alkalilauge, ein weitgehend konstanter pH-Wert von etwa 0,5-5, insbesondere etwa 1,5-2,5 eingehalten. Sobald die gewünschte Schichtdicke der $TiO_2$-Fällung erreicht ist, wird die Zugabe der Titansalzlösung und der Base gestoppt.

Dieses, auch als Titrationsverfahren bezeichnete Verfahren zeichnet sich dadurch aus, daß ein Überschuß an Titansalz vermieden wird. Das wird dadurch erreicht, daß man pro Zeiteinheit nur eine solche Menge der Hydrolyse zuführt, wie sie für eine gleichmäßige Beschichtung mit dem hydratisierten $TiO_2$ erforderlich ist und wie pro Zeiteinheit von der verfügbaren Oberfläche der zu beschichtenden Teilchen aufgenommen werden kann. Es entstehen deshalb keine hydratisierten Titandioxidteilchen, die nicht auf der zu beschichtenden Oberfläche niedergeschlagen sind.

Für das Aufbringen der Siliziumdioxidschichten ist folgendes Verfahren anzuwenden: Zu einer auf etwa 50-100 °C, insbesondere 70-80 °C erhitzten Suspension des zu beschichtenden Materials wird eine Natronwasserglaslösung zudosiert. Durch gleichzeitige Zugabe von 10%iger Salzsäure wird der pH-Wert bei 4 bis 10, vorzugsweise bei 6,5 bis 8,5 konstant gehalten. Nach Zugabe der Wasserglaslösung wird noch 30 min nachgerührt.

Es ist zusätzlich möglich, die Pulverfarbe des Pigmentes zu verändern, durch Aufbringen weiterer Schichten, wie z.B. farbige Metalloxide oder Berliner Blau, Verbindungen der Übergangsmetalle wie z.B. Fe, Cu, Ni, Co, Cr oder organische Verbindungen wie Farbstoffe oder Farblacke.

Die naßchemische Erzeugung von 2 oder mehr Interferenzschichten unterschiedlicher Brechzahl mit genau definierten Dicken auf feinteiligen, plättchenförmigen Substraten ist in wäßrigem Medium mit rein anorganischen Ausgangsstoffen bisher nicht bekannt.

Es ist weiterhin möglich, das fertige Pigment einer Nachbeschichtung oder Nachbehandlung zu unterziehen, die die Licht-, Wetter- und chemische Stabilität weiter erhöht, oder die Handhabung des Pigments, insbesondere die Einarbeitung in unterschiedliche Medien, erleichtert. Als Nachbeschichtungen bzw. Nachbehandlungen kommen beispielsweise die in den DE-PS 22 15 191, DE-OS 31 51 354, DE-OS 32 35 017 oder DE-OS 33 34 598 beschriebenen Verfahren in Frage.

Die zusätzlich aufgebrachten Stoffe machen nur etwa 0,1 bis 5 Gew.-%, vorzugsweise etwa 0,5 bis 3 Gew.-%, des gesamten Pigmentes aus.

Das erfindungsgemäße Pigment kann noch zusätzlich mit schwerlöslichen, fest haftenden anorganischen oder organischen Farbmitteln beschichtet werden. Bevorzugt werden Farblacke und insbesondere Aluminiumfarblacke verwendet. Dazu wird eine Aluminiumhydroxidschicht aufgefällt, die in einem zweiten Schritt mit einem Farblack verlackt wird. Das Verfahren ist in DE 24 29 762 und DE 29 28 287 näher beschrieben.

Bevorzugt ist auch eine zusätzliche Beschichtung mit Komplexsalzpigmenten, insbesondere Cyanoferratkomplexen, wie zum Beispiel Berliner Blau und Turnbulls Blau, wie sie in EP 0 141 173 und DE 23 13 332 beschrieben ist.

Das erfindungsgemäße Pigment kann auch mit organischen Farbstoffen und insbesondere mit Phthalocyanin- oder Metallphthalocyanin- und/oder Indanthrenfarbstoffen nach DE 40 09 567 beschichtet werden. Dazu wird eine Suspension des Pigmentes in einer Lösung des Farbstoffes hergestellt und diese dann mit einem Lösungsmittel zusammengebracht, in welchem der Farbstoff schwer löslich oder unlöslich ist.

Weiterhin können auch Metallchalkogenide bzw. Metalchalkogenidhydrate und Ruß für eine zusätzliche Beschichtung eingesetzt werden.

Das Pigment kann auf übliche Weise zur Pigmentierung von Lacken, Druckfarben, Kunststoffen, Kosmetika und Glasuren für Keramiken und Gläser verwendet werden. Bevorzugt wird es zur Pigmentierung von Agrarfolien eingesetzt.

Agrarfolien werden vielfach mit Pigmenten ausgerüstet, um die Infrarotstrahlung der Sonne fern zu halten und so eine übermäßige Erwärmung, z.B. eines Gewächshauses, zu verhindern.

Bei den bisher in Agrarfolien verwendeten Pigmenten handelt es sich fast ausschließlich um Farbpigmente. Daher absorbieren bzw. reflektieren sie einen wesentlichen Anteil des sichtbaren Lichtes, das aber die unter der Folie lebenden Pflanzen für ihr Wachstum benötigen. Daher wirken sich die bisher in Agrarfolien verwendeten Pigmente negativ auf das Wachstumsverhalten der Pflanzen aus.

Eine Aufgabe der vorliegenden Erfindung war es daher, ein Interferenzpigment zur Verfügung zu stellen, das im sichtbaren Bereich des Lichtes eine hohe Durchlässigkeit und im NIR-Bereich eine hohe Reflektivität besitzt. Derartige Pigmente können in ihren Eigenschaften auch in der Weise eingestellt werden, daß sie andere bzw. zusätzliche Funktionen, wie z.B. die gezielte Beeinflussung der Morphogenese von Pflanzen, besitzen.

Die folgenden Beispiele sollen die Erfindung näher erläutern, ohne sie zu begrenzen.

**Beispiel 1**

**3-Schicht-System mit dünner $SiO_2$-Schicht**

1) Erste $TiO_2$-Schicht:

150 g Glimmer (Teilchengröße 10-40 $\mu$m) werden in 2 l vollentsalztem Wasser (VE-Wasser) suspendiert und auf 75 °C erhitzt. Zu dieser Suspension werden 175 ml einer wäßrigen TiCl$_4$-Lösung (400 g TiCl$_4$) innerhalb von 60 min hinzudosiert. Der pH-Wert wird während der gesamten Zugabe mit 32%iger NaOH-Lösung bei 2,2 konstant gehalten. Nach beendeter Zugabe wird zur Vervollständigung der Fällung 30 min bei 75 °C nachgerührt.

2) SiO$_2$-Schicht:

Der pH-Wert der Suspension wird mit NaOH-Lösung auf 7,5 erhöht und innerhalb von 90 min werden 250 ml einer NatronwasserglasLösung (125 g SiO$_2$/l) bei 75 °C zudosiert. Dabei wird der pH-Wert mit 10%iger Salzsäure konstant gehalten. Nach beendeter Zugabe wird zur Vervollständigung der Fällung erneut 30 min bei 75 °C nachgerührt.

3) Zweite TiO$_2$-Schicht:

Der pH-Wert wird mit 10%iger Salzsäure wieder auf 2,2 abgesenkt und erneut TiO$_2$ aus weiteren 175 ml TiCl$_4$-Lösung, wie in Schritt 1) beschrieben, aufgefällt.

Anschließend läßt man auf Raumtemperatur abkühlen, filtriert das erhaltene Pigment ab, wäscht mit VE-Wasser salzfrei und trocknet bei 110 °C. Danach wird das Pigment 30 min bei 850 °C geglüht.

Das so erhaltene Pigment zeichnet sich durch eine intensivere blaue Interferenzfarbe und höhere Transparenz als vergleichbare reine TiO$_2$-Glimmer-Pigmente aus.

**Beispiel 2**

**3-Schicht-System mit dicker SiO$_2$-Schicht**

1) Erste TiO$_2$-Schicht:

150 g Glimmer (Teilchengröße 10-40 $\mu$m) werden in 2 l (VE-Wasser) suspendiert und auf 75 °C erhitzt. Zu dieser Suspension werden 300 ml einer wäßrigen TiCl$_4$-Lösung (400 g TiCl$_4$/l) innerhalb von 100 min hinzudosiert. Der pH-Wert wird während der gesamten Zugabe mit 32%iger NaOH-Lösung bei 2,2 konstant gehalten. Nach beendeter Zugabe wird zur Vervollständigung der Fällung noch 30 min bei 75 °C nachgerührt.

2) SiO$_2$-Schicht:

Der pH-Wert der Suspension wird mit NaOH-Lösung auf 7,5 erhöht und innerhalb von 7,5 h werden 1350 ml einer NatronwasserglasLösung (125 g SiO$_2$/l) bei 75 °C zudosiert. Dabei wird der pH-Wert mit 10%iger Salzsäure konstant gehalten. Nach beendeter Zugabe wird zur Vervollständigung der Fällung erneut 30 min bei 75 °C nachgerührt.

3) Zweite TiO$_2$-Schicht:

Der pH-Wert wird wieder auf 2,2 abgesenkt und erneut TiO$_2$ aus weiteren 250 ml TiCl$_4$-Lösung, wie in Schritt 1) beschrieben, aufgefällt.

Anschließend läßt man auf Raumtemperarur abkühlen, filtriert das erhaltene Pigment ab, wäscht mit VE-Wasser salzfrei und trocknet bei 110 °C. Danach wird das Pigment 30 min bei 850 °C geglüht.

Das so erhaltene Pigment zeigt bei senkrechter Betrachtung eine intensive blaugrüne Interferenzfarbe, die beim Abkippen über violett in rot übergeht.

**Beispiel 4**

**Mehrschichtsystem aus 5 alternierenden TiO$_2$- und SiO$_2$-Schichten**

Wie in Beispiel 1 beschrieben, wird Glimmer mit TiO$_2$, SiO$_2$ und TiO$_2$ beschichtet. Danach werden erneut eine SiO$_2$-Schicht und eine abschließende TiO$_2$-Schicht aufgebracht. Die Aufarbeitung erfolgt wie oben beschrieben.

Das erhaltene Pigment hat eine klarere blaue Interferenzfarbe und höhere Tranzparenz als dasjenige von Beispiel 1.

**Beispiel 5**

**3-Schicht-System mit hoher Transparenz im sichtbaren Bereich und hoher Reflektion im nahen Infrarot-Bereich**

100 g Glimmer (Teilchengröße 10-60 $\mu$m) werden in 2 l vollentsalztem Wasser suspendiert und unter starkem Rühren auf 80 °C erhitzt. Zu dieser Mischung wird bei pH 2,0 eine Lösung von 3 g SnCl$_4$ x 5 H$_2$O und 10 ml Salzsäure (37 %) in 90 ml vollentsalztem Wasser mit einer Geschwindigkeit von 4 ml/min. zudosiert. Anschließend wird bei einem pH-Wert von 1,8 eine Menge von 481 ml TiCl$_4$-Lösung (400 g TiCl$_4$/l) mit einer Geschwindigkeit von 2 ml/min. zudosiert. Danach wird der pH-Wert mit Natronlauge (32 %) auf 7,5 eingestellt und bei diesem pH-Wert eine Lösung von 230 ml Natronwasserglaslösung (Fa. Merck; Best.-Nr. 5621) in 314 ml vollentsalztem Wasser mit einer Geschwindigkeit von 2 ml/min. zudosiert. Der pH-Wert wird dabei mit Salzsäure (10 %) konstant gehalten. Anschließend wird bei pH 2,0 eine Lösung von 3 g $SnCl_4$ x 5 $H_2O$ und 10 ml Salzsäure (32 %) in 90 ml vollentsalztem Wasser mit einer Geschwindigkeit von 4 ml/min, zudosiert. Anschließend werden bei einem pH-Wert von 1,8 481 ml $TiCl_4$-Lösung (400 g $TiCl_4$/l) mit einer Geschwindigkeit von 2 ml/min. zudosiert.

Der pH-Wert wird bei der Zugabe der $SnCl_4$ x 5 $H_2O$-Lösungen und $TiCl_4$-Lösungen jeweils mit NaOH-Lösung (32 %) konstant gehalten.

Zur Aufarbeitung wird das Pigment abfiltriert, mit 20 l vollentsalztem Wasser gewaschen, bei 110 °C getrocknet und 30 min. bei 850 °C geglüht.

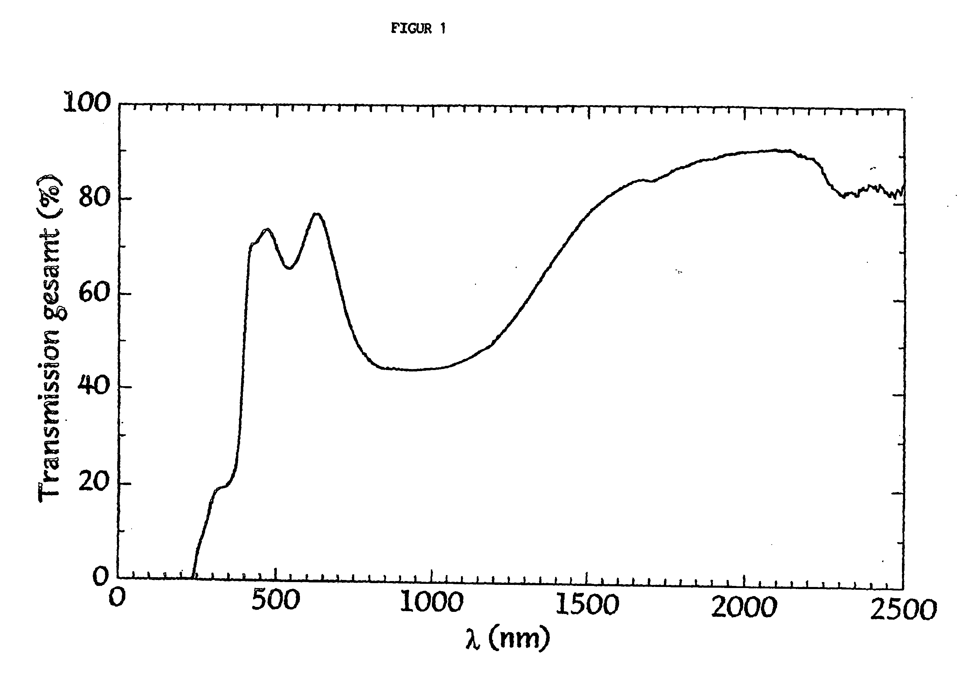

Von diesem Pigment wurde ein Lackfilm hergestellt, dessen Transmissionsspektrum in Figur 1 wiedergegeben ist. Das Pigment zeichnet sich durch eine sehr gute Transparenz im sichtbaren Bereich des Lichtes und eine sehr hohe Reflektion im nahen Infrarot-Bereich aus, die mit herkömmlichen Interferenzpigmenten nicht zu erreichen sind. Daher ist dieses Pigment insbesondere für die Anwendung in Agrarfolien geeignet.

**Beispiel 6**

**Pigment mit hoher Winkelabhängigkeit der Farbe**

100 g eines $TiO_2$-Glimmer-Pigmentes (Teilchengröße 10-60 $\mu$m, 35 % $TiO_2$) werden in 2 l vollentsalztem Wasser supendiert und unter starkem Rühren auf 80 °C erhitzt. Anschließend wird der pH-Wert mit Natronlauge (32 %) auf 7,5 eingestellt und bei diesem pH-Wert eine Lösung von 296 ml Natronwasserglaslösung (Fa. Merck; Best.-Nr. 5621) in 300 ml vollentsalztem Wasser mit einer Geschwindigkeit von 2 ml/min. zudosiert. Der pH-Wert wird dabei mit Salzsäure (10 %) konstant gehalten. Danach wird zu dieser Mischung bei pH 2,0 eine Lösung von 3 g $SnCl_4$ x 5 $H_2O$ und 10 ml Salzsäure (37 %) in 90 ml vollentsalztem Wasser mit einer Geschwindigkeit von 4 ml/min. zudosiert. Anschließend wird bei pH-Wert 1,8 eine Menge von 238 ml $TiCl_4$-Lösung (400 g $TiCl_4$/l) mit einer Geschwindigkeit von 2 ml/min. zudosiert.

Der pH-Wert wird bei der Zugabe der $SnCl_4$ x 5 $H_2O$-Lösung und der $TiCl_4$-Lösung jeweils mit NaOH-Lösung (32 %) konstant gehalten.

Zur Aufarbeitung wird das Pigment abfiltriert, mit 20 l vollentsalztem Wasser gewaschen, bei 110 °C getrocknet und 30 min. bei 850 °C geglüht.

Das geglühte Pigment wird in einen Klarlack eingerührt (Konzentration 1,7 %) und auf einer Schwarz/Weiß-Karte ausgestrichen.

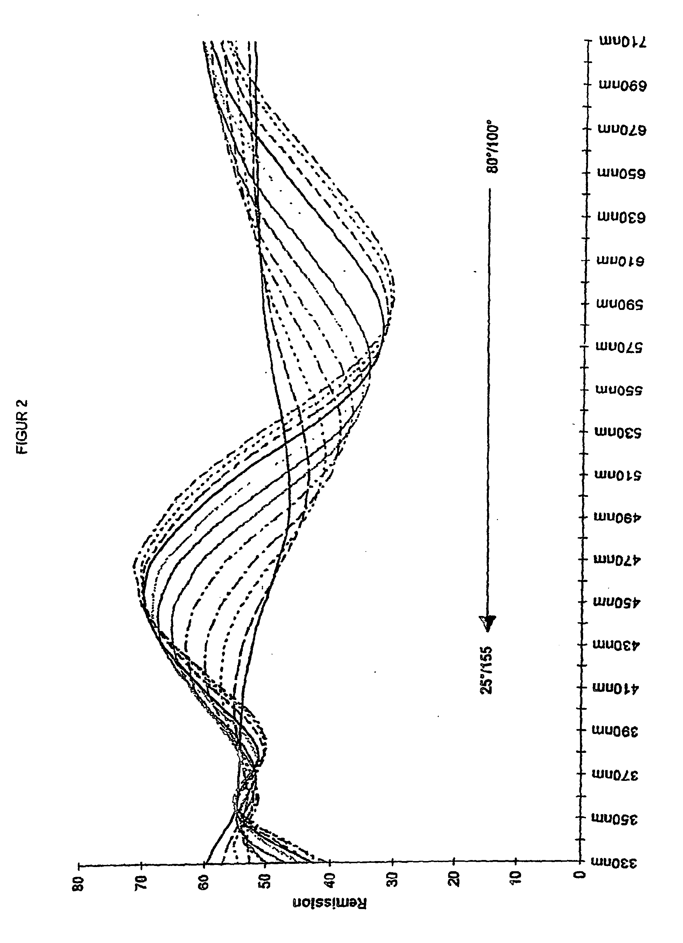

Insbesondere auf der Schwarzkarte tritt die Interferenzfarbe des Pigmentes deutlich hervor. Beim Abkippen der Karte von einem steilen zu einem flachen Betrachtungswinkel ändert sich die reflektierte Farbe von intensiv Blau nach intensiv Violett. Das Reflektionsspektrum der Schwarzkarte wurde unter verschiedenen steilen (80°/100°) und flachen (25°/155°) Beobachtungswinkeln gemessen. Die Reflektionskurven sind in Figur 2 wiedergegeben.

**Beispiel 7**

**3-Schichtsystem mit $SiO_2$-Plättchen als Trägermaterial**

1) Erste $TiO_2$-Schicht:

100 g $SiO_2$-Flakes (Teilchengröße 20-70 um) werden in 1,5 l vollentsalztem Wasser suspendiert und auf 75 °C erhitzt. Zu dieser Suspension werden 160 ml einer wäßrigen $TiCl_4$-Lösung (400 g $TiCl_4$/l) innerhalb von 90 min hinzudosiert. Der pH-Wert wird während der gesamten Zugabe mit 32%iger NaOH-Lösung bei 2,2 konstant gehalten. Nach beendeter Zugabe wird zur Vervollständigung der Fällung noch 30 min bei 75 °C nachgerührt.

2) $SiO_2$-Schicht:

Der pH-Wert der Suspension wird mit NaOH-Lösung auf 7,5 erhöht und innerhalb von 3,5 h werden 720 ml einer NatronwasserglasLösung (125 g $SiO_2$/l) bei 75 °C zudosiert. Dabei wird der pH-Wert mit 10%iger Salzsäure konstant gehalten. Nach beendeter Zugabe wird zur Vervollständigung der Fällung erneut 30 min bei 70 °C nachgerührt.

3) Zweite $TiO_2$-Schicht:

Der pH-Wert wird wieder auf 2,2 abgesenkt und erneut $TiO_2$ aus weiteren 235 ml $TiCl_4$-Lösung, wie in Schritt 1) beschrieben, aufgefällt.

Anschließend läßt man auf Raumtemperatur abkühlen, filtriert das erhaltene Pigment ab, wäscht mit vollentsalztem Wasser salzfrei und trocknet bei 110 °C. Danach wird das Pigment 30 min bei 850 °C geglüht. Das so erhaltene Pigment zeigt bei senkrechter Betrachtung eine leuchtende gelb-grüne Interferenzfarbe, die beim Abkippen über blau-grün nach dunkel violett übergeht.

**Patentansprüche**

1. Mehrschichtiges Interferenzpigment, bestehend aus einem transparenten Trägermaterial, das mit alternierenden Schichten von Metalloxiden mit niedriger und hoher Brechzahl beschichtet ist, wobei die Differenz der Brechzahlen mindestens 0,1 beträgt, erhältlich durch alternierende Beschichtung des transparenten Trägermaterials mit einem Metalloxid ohne absorbierende Eigenschaften mit hoher Brechzahl und einem Metalloxid mit niedriger Brechzahl im Naßverfahren durch Hydrolyse der entsprechenden, wasserlöslichen, anorganischen Metallverbindungen, Abtrennung, Trocknung und gegebenenfalls Kalzination des erhaltenen Pigmentes, wobei zumindest die Schichtfolge hochbrechendes Metalloxid / niedrigbrechendes Metalloxid /hochbrechendes Metalloxid enthalten ist und die Auffällung des jeweiligen Metalloxids durch gleichzeitige langsame Zugabe einer Metallsalzlösung und einer Base bzw. Säure zur Konstanthaltung eines zur Hydrolyse und Ausfällung geeigneten pH-Wertes zu einer Suspension des Trägermaterials erfolgt ist, und wobei die Dicke der einzelnen Metalloxidschichten, unabhängig von ihrer Brechzahl, 50 - 300 nm beträgt.

2. Interferenzpigment nach Anspruch 1, **dadurch gekennzeichnet, daß** das transparente Trägermaterial Glimmer, ein anderes Schichtsilikat, Glasplättchen, $PbCO_3$ x $Pb(OH)_2$, BiOCl oder plättchenförmiges $SiO_2$ ist.

3. Interferenzpigment nach den Ansprüchen 1 und 2, **dadurch gekennzeichnet, daß** das Oxid mit hoher Brechzahl $TiO_2$, $ZrO_2$, ZnO oder ein Gemisch aus diesen Oxiden ist.

4. Interferenzpigment nach mindestens einem der Ansprüche 1 bis 3, **dadurch gekennzeichnet, daß** das Metalloxid mit niedriger Brechzahl $SiO_2$, $Al_2O_3$, AlOOH, $B_2O_3$ oder eine Mischung daraus ist, wobei gegebenenfalls Alkali- oder Erdalkalioxide als zusätzliche Bestandteile enthalten sein können.

5. Verfahren zur Herstellung des Interferenzpigmentes nach den Ansprüchen 1 bis 4, **dadurch gekennzeichnet, daß** das transparente Trägermaterial in Wasser suspendiert und abwechselnd mit einem Metalloxidhydrat mit hoher Brechzahl ohne absorbierende Eigenschaften und einem Metalloxidhydrat mit niedriger Brechzahl durch langsame Zugabe und Hydrolyse der entsprechenden, anorganischen, wasserlöslichen Metallverbindungen beschichtet wird, wobei durch gleichzeitige Zugabe von Säure oder Base der für die Fällung des jeweiligen Metalloxidhydrates notwendige pH-Wert eingestellt und konstant gehalten wird und wobei zumindest die Schichtfolge hochbrechendes Metalloxid / niedrigbrechendes Metalloxid / hochbrechendes Metalloxid aufgefällt wird und anschließend das beschichtete Trägermaterial aus der wäßrigen Suspension abgetrennt, getrocknet und gegebenenfalls kalziniert wird.

6. Verfahren nach Anspruch 5, **dadurch gekennzeichnet, daß** als transparentes Trägermaterial Glimmer, ein anderes Schichtsilikat, $PbCO_3$6 x $Pb(OH)_2$, BiOCl oder plättchenförmiges $SiO_2$ eingesetzt wird.

7. Verfahren nach den Ansprüchen 5 und 6, **dadurch gekennzeichnet, daß** das Metalloxid mit hoher Brechzahl $TiO_2$, $ZrO_2$ oder ZnO ist.

8. Verfahren nach mindestens einem der Ansprüche 5 bis 7, **dadurch gekennzeichnet, daß** das Metalloxid mit niedriger Brechzahl $SiO_2$, $Al_2O_3$, AlOOH, $B_2O_3$ oder eine Mischung daraus ist, wobei gegebenenfalls Alkali- und Erdalkalioxide als zusätzliche Bestandteile enthalten sein können.

9. Verwendung der Pigmente nach den Ansprüchen 1 bis 4 zur Pigmentierung von Lacken, Druckfarben, Kunststoffe, Kosmetika, Glasuren für Keramiken und Gläser.

10. Verwendung nach Anspruch 9, **dadurch gekennzeichnet, daß** die Pigmente als Mischungen mit handelsüblichen Pigmenten eingesetzt werden.

11. Verwendung der Pigmente nach den Ansprüchen 1 bis 4 zur Lasermarkierung von Kunststoffen.

12. Lacke, Druckfarben, Kunststoffe, Kosmetika, Keramiken und Gläser, welche mit einem Pigment nach den Ansprüchen 1 bis 4 pigmentiert sind.

13. Lasermarkierbare Kunststoffe enthaltend Pigmente nach den Ansprüchen 1 bis 4.

**Claims**

1. Multilayered interference pigment, consisting of a transparent support material which is coated with alternating layers of metal oxides of low and high refractive index, where the difference between the refractive indices is at least 0.1, obtainable by alternate coating of the transparent support material with a metal oxide of high refractive index with no absorbent properties and a metal oxide of low refractive index by the wet process by hydrolysis of the corresponding water-soluble, inorganic metal compounds, separation, drying and optionally calcination of the resultant pigment, where at least the layer sequence high-refractive-index metal oxide / low-refractive-index metal oxide / high-refractive-index metal oxide is present and the deposition of the respective metal oxide has been carried out by simultaneous slow addition of a metal-salt solution and a base or acid in order to keep a constant pH which is suitable for the hydrolysis and precipitation to a suspension of the support material, and where the thickness of the individual metal-oxide layers, irrespective of their refractive index, is 50-300 nm.

2. Interference pigment according to Claim 1, **characterised in that** the transparent support material is mica, another phyllosilicate, a glass flake, $PbCO_3$ x $Pb(OH)_2$, BiOCl or flake-form $SiO_2$.

3. Interference pigment according to Claims 1 and 2, **characterised in that** the oxide of high refractive index is $TiO_2$, $ZrO_2$, ZnO or a mixture of these oxides.

4. Interference pigment according to at least one of Claims 1 to 3, **characterised in that** the metal oxide of low refractive index is $SiO_2$, $Al_2O_3$, AlOOH, $B_2O_3$ or a mixture thereof, where alkali metal or alkaline-earth metal oxides may optionally be present as additional constituents.

5. Process for the preparation of the interference pigment according to Claims 1 to 4, **characterised in that** the transparent support material is suspended in water and coated alternately with a metal oxide hydrate of high refractive index with no absorbent properties and a metal oxide hydrate of low refractive index by slow addition and hydrolysis of the corresponding inorganic, water-soluble metal compounds, where the pH necessary for the precipitation of the respective metal oxide hydrate is established and kept constant by simultaneous addition of acid or base and where at least the layer sequence high-refractive-index metal oxide / low-refractive-index metal oxide / high-refractive-index metal oxide is deposited and the coated support material is subsequently separated off from the aqueous suspension, dried and optionally calcined.

6. Process according to Claim 5, **characterised in that** the transparent support material employed is mica, another phyllosilicate, $PbCO_3$ x $Pb(OH)_2$, BiOCl or flake-form $SiO_2$.

7. Process according to Claims 5 and 6, **characterised in that** the metal oxide of high refractive index is $TiO_2$, $ZrO_2$ or ZnO.

8. Process according to at least one of Claims 5 to 7, **characterised in that** the metal oxide of low refractive index is $SiO_2$, $Al_2O_3$, AlOOH, $B_2O_3$ or a mixture thereof, where alkali metal and alkaline-earth metal oxides may optionally be present as additional constituents.

9. Use of the pigments according to Claims 1 to 4 for the pigmentation of surface coatings, printing inks, plastics, cosmetics, glazes for ceramics and glasses.

10. Use according to Claim 9, **characterised in that** the pigments are employed as mixtures with commercially available pigments.

11. Use of the pigments according to Claims 1 to 4 for the laser marking of plastics.

12. Surface coatings, printing inks, plastics, cosmetics, ceramics and glasses which are pigmented with a pigment according to Claims 1 to 4.

13. Laser-markable plastics comprising pigments according to Claims 1 to 4.

**Revendications**

1. Pigment d'interférence multi-couches, constitué d'un matériau support transparent qui est revêtu de couches alternées d'oxydes métalliques d'indice de réfraction élevé et bas, où la différence entre les indices de réfraction est d'au moins 0,1, pouvant être obtenu par le revêtement alterné du matériau support transparent par un oxyde métallique d'indice de réfraction élevé n'ayant pas de propriétés absorbantes et un oxyde métallique d'indice de réfraction bas par le procédé par voie humide par l'hydrolyse des composés métalliques minéraux hydrosolubles correspondants, la séparation, le séchage et éventuellement la calcination du pigment résultant, où au moins la séquence de couches oxyde métallique d'indice de réfraction élevé /oxyde métallique d'indice de réfraction bas / oxyde métallique d'indice de réfraction élevé est présente et le dépôt de l'oxyde métallique respectif a été effectué par addition lente simultanée d'une solution de sel métallique et d'une base ou d'un acide afin de maintenir un pH constant qui est convenable pour l'hydrolyse et la précipitation sur une suspension du matériau support, et où l'épaisseur des couches d'oxyde métallique individuelles, indépendamment de leur indice de réfraction, est de 50-300 nm.

2. Pigment d'interférence selon la revendication 1, **caractérisé en ce que** le matériau support transparent est le mica, un autre phyllosilicate, une paillette de verre, $PbCO_3$ x $Pb(OH)_2$, BiOCl ou $SiO_2$ sous forme de paillettes.

3. Pigment d'interférence selon les revendications 1 et 2, **caractérisé en ce que** l'oxyde d'indice de réfraction élevé est $TiO_2$, $ZrO_2$, ZnO ou un mélange de ces oxydes.

4. Pigment d'interférence selon au moins l'une des revendications 1 à 3, **caractérisé en ce que** l'oxyde métallique d'indice de réfraction bas est $SiO_2$, $Al_2O_3$, AlOOH, $B_2O_3$ ou un mélange de ceux-ci, où des oxydes de métaux alcalins ou de métaux alcalino-terreux peuvent éventuellement être présents comme constituants supplémentaires.

**5.** Procédé de préparation du pigment d'interférence selon les revendications 1 à 4, **caractérisé en ce que** le matériau support transparent est mis en suspension dans de l'eau et revêtu en alternance avec un oxyde métallique hydraté d'indice de réfraction élevé n'ayant pas de propriétés absorbantes et un oxyde métallique hydraté d'indice de réfraction bas par addition lente et hydrolyse des composés métalliques hydrosolubles minéraux correspondants, où le pH nécessaire à la précipitation de l'oxyde métallique hydraté respectif est établi et maintenu constant par l'addition simultanée d'acide ou de base et où au moins la séquence de couches oxyde métallique d'indice de réfraction élevé / oxyde métallique d'indice de réfraction bas / oxyde métallique d'indice de réfraction élevé est déposée et le matériau support revêtu est ensuite séparé de la suspension aqueuse, séché et éventuellement calciné.

**6.** Procédé selon la revendication 5, **caractérisé en ce que** le matériau support transparent employé est le mica, un autre phyllosilicate, $PbCO_3 \times Pb(OH)_2$, BiOCl ou $SiO_2$ sous forme de paillettes.

**7.** Procédé selon les revendications 5 et 6, **caractérisé en ce que** l'oxyde métallique d'indice de réfraction élevé est $TiO_2$, $ZrO_2$ ou ZnO.

**8.** Procédé selon au moins l'une des revendications 5 à 7, **caractérisé en ce que** l'oxyde métallique d'indice de réfraction bas est $SiO_2$, $Al_2O_3$, AlOOH, $B_2O_3$ ou un mélange de ceux-ci, où des oxydes de métaux alcalins ou de métaux alcalino-terreux peuvent éventuellement être présents comme constituants supplémentaires.

**9.** Utilisation des pigments selon les revendications 1 à 4 pour la pigmentation de revêtements de surface, d'encres d'impression, de matières plastiques, de produits cosmétiques, de vernissage de céramiques et de verres.

**10.** Utilisation selon la revendication 9, **caractérisée en ce que** les pigments sont employés comme mélanges avec des pigments disponibles dans le commerce.

**11.** Utilisation des pigments selon les revendications 1 à 4 pour la marquage au laser de matières plastiques.

**12.** Revêtements de surface, encres d'impression, matières plastiques, produits cosmétiques, céramiques et verres qui sont pigmentés par un pigment selon les revendications 1 à 4.

**13.** Matières plastiques pouvant être marquées au laser, comprenant des pigments selon les revendications 1 à 4.

FIGUR 1

*Transmission gesamt (%)* vs *λ (nm)*

FIGUR 2

**IN DER BESCHREIBUNG AUFGEFÜHRTE DOKUMENTE**

**In der Beschreibung aufgeführte Patentdokumente**

- US 4434010 A
- JP H7759 A
- EP 0708154 A
- JP 7246366 A
- JP 2016168 A
- WO 9308237 A
- DE 1467468
- DE 1959988
- DE 2009566
- DE 2214545
- DE 2215191
- DE 2244298
- DE 2313331
- DE 2522572
- DE 3137808
- DE 3137809
- DE 3151343
- DE 3151354
- DE 3151355
- DE 3211602
- DE 3235017
- EP 0045851 A
- EP 0106235 A
- US 3553001 A
- DE 2215191 C
- DE 3151354 A
- DE 3235017 A
- DE 3334598 A
- DE 2429762
- DE 2928287
- EP 0141173 A
- DE 2313332
- DE 4009567